# EUROPÄISCHE PATENTSCHRIFT

(11) **EP 2 627 411 B1**
(45) Veröffentlichungstag und Bekanntmachung des Hinweises auf die Patenterteilung: **07.12.2016**
(21) Anmeldenummer: 11767433.3
(22) Anmeldetag: 07.10.2011
(51) Int. Cl.: A61N 5/10

(54) **BESTRAHLUNGSPLANUNG MIT INHOMOGENEM TEILCHENSTRAHL**
RADIATION PLANNING FOR RADIATION HAVING AN INHOMOGENEOUS PARTICLE BEAM
PLANIFICATION D'IRRADIATION AU MOYEN D'UN FAISCEAU DE PARTICULES HÉTÉROGÈNE

(30) Priorität: 12.10.2010 DE 102010048232
(43) Veröffentlichungstag der Anmeldung: 21.08.2013
(73) Patentinhaber: GSI Helmholtzzentrum für Schwerionenforschung GmbH, 64291 Darmstadt (DE)
(72) Erfinder: BERT, Christoph, 63741 Aschaffenburg (DE); NGUYEN, Johnny, 64295 Darmstadt (DE)
(86) Internationale Anmeldenummer: PCT/EP2011/067536
(87) Internationale Veröffentlichungsnummer: WO 2012/049081

(56) Entgegenhaltungen:
- EP-A1- 0 212 793
- WO-A1-2008/106492
- SZYMANOWSKI H ET AL: "Two-dimensional pencil beam scaling: an improved proton dose algorithm for heterogeneous media", PHYSICS IN MEDICINE AND BIOLOGY, INSTITUTE OF PHYSICS PUBLISHING, BRISTOL GB, vol. 47, 5 September 2002 (2002-09-05), pages 3313-3330, XP002538781, ISSN: 0031-9155, DOI: 10.1088/0031-9155/47/18/304

## Beschreibung

Die Erfindung betrifft ein Verfahren zur Erstellung einer Bestrahlungsplanung zur Bestrahlung eines zumindest zeitweise und/oder zumindest bereichsweise bewegten Zielvolumenbereichs mit einer Strahlerzeugungsvorrichtung, bei dem ein Dosiseintrag des Bestrahlungs-Strahls der Strahlerzeugungsvorrichtung in einem Zielvolumenbereich berücksichtigt wird. Weiterhin betrifft die Erfindung ein Verfahren zur Ansteuerung einer Strahlungserzeugungsvorrichtung zur Applizierung einer ortsaufgelösten Strahlendosis, ausgenommen zur Behandlung von Menschen und Tieren, in zumindest einem zumindest zeitweise und/oder zumindest bereichsweise bewegten Zielvolumenbereich unter Verwendung der Strahlerzeugungsvorrichtung. Weiterhin betrifft die Erfindung eine Vorrichtung zur Erstellung einer Bestrahlungsplanung. Schließlich betrifft die Erfindung eine Vorrichtung zur Ansteuerung zumindest einer Strahlerzeugungsvorrichtung und/oder eine Strahlerzeugungsvorrichtung.

Heutzutage kommt es in unterschiedlichsten Bereichen der Technik zu einer Bestrahlung von Gegenständen. Je nach konkretem Einsatzerfordernis werden dabei unterschiedlichste Bestrahlungsverfahren sowie verschiedene Strahlungsarten (beispielsweise Photonenstrahlung, Teilchenstrahlung usw.) verwendet.

Je nach Einsatzgebiet werden die Gegenstände flächig beziehungsweise räumlich homogen bestrahlt, beziehungsweise positionsabhängig unterschiedlich stark bestrahlt. Wenn beispielsweise Materialien gehärtet oder in sonstiger Weise bezüglich ihrer Materialeigenschaften verändert werden sollen, so ist oftmals ein möglichst homogener Strahlungseintrag erwünscht. Auch im Bereich der Lebensmitteltechnik werden heutzutage zum Teil Lebensmittel radioaktiv bestrahlt, um diese haltbarer zu machen. Eine in Abhängigkeit von der Position unterschiedlich starke Bestrahlung (strukturierte Bestrahlung) ist dagegen beispielsweise bei der Strukturierung von Mikroprozessoren oder sonstigen Mikrostrukturen beziehungsweise Nanostrukturen erforderlich. Eine derartige Strukturierung kann beispielsweise unter Verwendung von elektromagnetischer Strahlung sowie bildgebender Masken realisiert werden. Auch eine im Rahmen eines Scan-Verfahrens eingebrachte Bestrahlung (beispielsweise Elektronenstrahlen, Protonenstrahlen, Schwerionenstrahlen) ist in manchen Gebieten üblich.

Eine besondere Art der strukturierten Bestrahlung besteht darin, dass nicht nur eine zweidimensional strukturierte Bestrahlung erfolgt, sondern sogar eine dreidimensional strukturierte Bestrahlung. Dadurch ist es beispielsweise möglich, im Inneren eines zu bestrahlenden Körpers liegende Volumenbereiche mit einem Strahlungseintrag zu versehen, während andere Volumenbereiche (insbesondere auch Oberflächenbereiche) nicht beziehungsweise nur wenig stark bestrahlt werden. Hierzu werden oftmals Hadronenstrahlen verwendet (insbesondere Protonenstrahlen und Schwerionenstrahlen), da derartige Partikel einen ausgeprägten Bragg-Peak aufweisen. Das heißt, dass sie den Hauptteil ihrer Energie in einem räumlich relativ eng begrenzten Bereich abgeben (typischerweise kurz bevor diese im Material "stecken bleiben").

Eine weitere Komplikation tritt auf, wenn der zu bestrahlende Körper (beziehungsweise bestimmte Volumenbereiche des zu bestrahlenden Körpers) nicht statisch/nicht unbewegt vorliegt, sondern sich zumindest zeitweise beziehungsweise zumindest bereichsweise bewegt. Eine Bewegung kann dabei nicht nur translatorisch relativ zu einem äußeren Koordinatensystem erfolgen, sondern auch in Form einer Verschiebung verschiedener Bereiche des zu bestrahlenden Körpers relativ zueinander (einschließlich Verdrehungen, Verformungen, Stauchungen und/oder Dehnungen) erfolgen.

Um derartige, (in sich) bewegte Körper bestrahlen zu können, werden so genannte 4D-Bestrahlungsverfahren (vierdimensionale Bestrahlungsverfahren) verwendet. Dabei handelt es sich schlussendlich um dreidimensionale Bestrahlungsverfahren, die zeitlich variiert werden (die Zeit also die Rolle einer vierten Dimension einnimmt). Beispiele für derartige Materialbearbeitungsverfahren gibt es im Bereich der Materialwissenschaften.

Ein Gebiet der Technik, auf dem sich in letzter Zeit dreidimensionale und vierdimensionale Bestrahlungsverfahren zunehmend verbreitet haben, liegt im Bereich der Medizintechnik. Hier ist es in der Regel erforderlich, bestimmte Volumenbereiche innerhalb eines Körpers (wie beispielsweise einen Tumor) zu bestrahlen. Unter Bestrahlung wird die Beaufschlagung des Volumenbereiches innerhalb des Körpers mit Strahlung, beispielsweise Teilchenstrahlung oder Photonenstrahlung, verstanden. Insbesondere ist es erforderlich, den bestimmten Volumenbereich mit einer möglichst hohen Dosis zu beaufschlagen, ohne den Körper (beispielsweise durch chirurgische Verfahren) öffnen zu müssen. Das umliegende Gewebe soll dabei möglichst wenig beziehungsweise vorzugsweise im Wesentlichen überhaupt nicht mit einer Bestrahlungsdosisdosis belastet werden. Dies gilt in besonderem Maße, wenn es sich bei dem umgebenden Gewebe um ein so genanntes kritisches Gewebe handelt, wie beispielsweise um ein empfindliches Organ (fachsprachlich als OAR für "organ at risk" bezeichnet). Hierbei kann es sich beispielsweise um das Rückenmark, um Blutgefäße oder Nervenknoten handeln.

In der europäischen Patentanmeldung EP 0 212 793 A1 wird ein Verfahren und eine Vorrichtung zur Bestrahlungsplanung bei Bestrahlungsverfahren vorgeschlagen. Die Bestrahlung erfolgt mithilfe von Röntgenstrahlung unter Verwendung von Strahl-formenden Masken. Bei der Berechnung der Bestrahlungsplanung wird nicht nur die primäre Röntgen-Strahlung berücksichtigt, sondern auch Sekundärstrahlung in Form von Compton-Streustrahlung, die durch Wechselwirkung der Röntgenstrahlung mit Patientengewebe in anderen Bereichen des Patientenkörpers erzeugt wird.

Insbesondere im Bereich der Medizintechnik sollten die angewendeten Verfahren aus nachvollziehbaren Gründen möglichst präzise und fehlerfrei arbeiten. Ein weiteres Problem im Bereich der Medizintechnik liegt darin, dass aufgrund unterschiedlicher Gegebenheiten (zum Beispiel unterschiedlicher Körperbau der Patienten, unterschiedliche Lage, unterschiedliche Größe, unterschiedliche Beschaffenheit der Tumore) eigentlich immer unterschiedliche Ausgangsbedingungen vorliegen. Das heißt, dass die Bestrahlung (Behandlung) des Patienten jeweils individuell erfolgen muss. Diese individuellen Eigenschaften werden nach dem derzeitigen Stand der Technik in aller Regel unter Verwendung so genannter Bestrahlungsplanungen berücksichtigt. Hierbei wird eine von einem Arzt vorgeschriebene Dosisverteilung ("verschriebene Dosis") für die unterschiedlichen Gewebebereiche in "maschinenlesbare Parameter" umgerechnet. Das heißt, es wird ein Parametersatz berechnet, der besagt, auf welche Weise der Patient mit dem Teilchenstrahl beaufschlagt werden muss, um die vom Arzt verschriebene Dosisverteilung bestmöglich zu erhalten. Für die Berechnung werden beispielsweise Strahllage, Strahlenergie, Strahleintrittsrichtung, Teilchenart und dergleichen berücksichtigt. Zusätzlich ist bei der Erstellung der Bestrahlungsplanung eine große Anzahl nicht-linearer und komplexer Zusammenhänge zu berücksichtigen. So ist zum Beispiel der (an sich unerwünschte) Dosiseintrag in das hinter dem Bragg-Peak, insbesondere jedoch in das vor dem Bragg-Peak liegende Gewebe zu berücksichtigen. Auch sind biologische Effekte zu berücksichtigen, die sich beispielsweise in der so genannten relativen biologischen Wirksamkeit manifestieren (die unter anderem von der Teilchenart, der Teilchenenergie und der Art des bestrahlten Gewebes abhängen kann) zu berücksichtigen.

Es hat sich gezeigt, dass die bislang üblicherweise eingesetzten Bestrahlungsplanungsverfahren bei bestimmten Rahmenbedingungen zu Problemen führen können. Insbesondere wurde beobachtet, dass in manchen Bereichen zum Teil nicht unerhebliche Fehldosierungen auftreten können. Darüber hinaus wurde beobachtet, dass zeitweise eine derart schnelle Teilchenstrahlanpassung erforderlich wird, dass diese technisch und/oder wirtschaftlich nicht beziehungsweise nur schwer zu erfüllen ist. Dies betrifft insbesondere eine Anpassung des Teilchenstrahls hinsichtlich der Eindringtiefe (Lage des Bragg-Peaks), da hierzu die Teilchenenergie nach dem Stand der Technik durch Repositionierung von passiven Energiemodulatoren (typischerweise dreieckförmige Keile aus einem energieabsorbierenden Material) bewegt werden müssen. Bei einer schnellen Variation der Energie des Teilchenstrahls müssen dabei große Massen sehr schnell und sehr präzise beschleunigt und anschließend wieder abgebremst werden. Dies ist unter Umständen nicht oder nur sehr schwer möglich.

Die erwähnten Probleme treten besonders häufig und/oder in einem deutlich größeren Ausmaß auf, wenn sich Teile des zu bestrahlenden Körpers (in sich) bewegen. Um derartige, sich bewegende Gegenstände/Körper bestrahlen zu können, werden insbesondere drei Ansätze verfolgt, nämlich so genannte Rescanning-Verfahren, so genannte Gating-Verfahren sowie so genannte Tracking-Verfahren.

Bei Rescanning-Verfahren wird der bestrahlte Bereich "künstlich vergrößert", so dass der an sich zu bestrahlende Volumenbereich des Körpers in im Wesentlichen sämtlichen Bewegungssituationen mit einer Dosis beaufschlagt wird (die in der Regel entsprechend der Anzahl der Bestrahlungsdurchgänge proportional reduziert ist). Nachteilig ist dabei, das ein relativ großer Anteil an an sich nicht zu bestrahlendem Material mit einer Dosis beaufschlagt wird. Bei so genannten Gating-Verfahren erfolgt eine Bestrahlung des Körpers nur dann, wenn sich dieser in einer bestimmten Bewegungsphase befindet. Zu anderen Zeiten erfolgt dagegen keine Bestrahlung. Nachteilig ist dabei, dass dies zu einer signifikanten Verlängerung der Bestrahlungszeit führt. Bei Tracking-Verfahren wird der Teilchenstrahl in Abhängigkeit von der Bewegung des zu bestrahlenden Volumenbereichs nachgeführt, insbesondere hinsichtlich seiner transversalen Lage (typischerweise unter Verwendung von Ablenkmagneten) und/oder seiner longitudinalen Lage (Lage des Bragg-Peaks; erfolgt durch Energievariation des Teilchenstrahls). Bei derartigen Bestrahlungsverfahren kann es unter anderem durch eine Verknüpfung von Bestrahlungsfortschritt und Bewegung des zu bestrahlenden Volumenbereichs zu Korrelationseffekten kommen, bei denen bestimmte Bereiche des Körpers signifikant überdosiert beziehungsweise unterdosiert werden können.

Es besteht somit nach wie vor ein Bedarf an verbesserten Methoden zur Bestrahlungsplanung beziehungsweise an verbesserten Vorrichtungen zur Applikation von Strahlung, welche insbesondere eine genauere Bestrahlung des zu bestrahlenden Körpers ermöglichen. Insbesondere besteht ein Bedarf an Verfahren und/oder Vorrichtungen, welche robuster gegenüber etwaigen Abweichungen von gemessenen und/oder angenommenen Normwerten sind.

Die Erfindung ist in den Ansprüchen definiert. Andere Ausführungsformen sind lediglich beispielhaft.

Es wird vorgeschlagen, ein Verfahren zur Erstellung einer Bestrahlungsplanung zur Bestrahlung eines zumindest zeitweise und/oder zumindest bereichsweise bewegten Zielvolumenbereichs mit einer Strahlerzeugungsvorrichtung, bei dem ein Dosiseintrag des Bestrahlungs-Strahls der Strahlerzeugungsvorrichtung in einem Zielvolumenbereich berücksichtigt wird, derart durchzuführen, dass bei der Erstellung der Bestrahlungsplanung zumindest bereichsweise zumindest eine Bestrahlungs-Strahl-Inhomogenität berücksichtigt wird, und wobei die Bestrahlung zumindest teilweise durch ein Scan-Verfahren erfolgt. Die Berücksichtigung des Dosiseintrags des Bestrahlungs-Strahls der Strahlungserzeugungsvorrichtung in einem Volumenbereich kann gegebenenfalls auch nur bereichsweise erfolgen. Die Erfinder haben herausgefunden, dass viele Ungenauigkeiten und Artefakte bei der Erstellung von Bestrahlungsplanungen darauf zurückzuführen sind, dass bislang Inhomogenitäten des Bestrahlungs-Strahls nicht beziehungsweise nur unzureichend berücksichtigt wurden. Denn bislang gehen viele Verfahren zur Berechnung einer Bestrahlungsplanung davon aus, dass der Strahl durch einen im Wesentlichen homogenen Strahl mit ausreichender Genauigkeit angenähert werden kann. Die Erfinder haben jedoch festgestellt, dass auch kleine Inhomogenitäten des Bestrahlung-Strahls zu signifikanten Dosisabweichungen in einem zu bestrahlenden Volumen und/oder sonstigen Problemen führen können (wie insbesondere stark schwankenden Ansteuerungsparametern für den Bestrahlung-Strahl). Unter einer Bestrahlungs-Strahl-Inhomogenität kann eine im Wesentlichen beliebige Abweichung (auch über die Zeit hinweg) verstanden werden. Beispielsweise kann es sich um eine zeitliche Variation des Strahldurchmessers, um eine (gegebenenfalls zeitlich variierende) nicht-runde (Nicht-sphärische) Teilchenstrahlform, um eine (gegebenenfalls zeitlich variierende) Energie-Dispersion des Teilchenstrahls und dergleichen handeln. Möglich ist es insbesondere, den Teilchenstrahl zu Berechnungszwecken in mehrere "gedachte" Teil-Bestrahlungs-Strahlen aufzuspalten, und diese jeweils einzeln im Rahmen der Bestrahlungsplanung zu berücksichtigen. Dabei ist es sinnvoll, dass zumindest ein Teil der Teil-Bestrahlungs-Strahlen unterschiedliche Eigenschaften aufweist, wie beispielsweise einen unterschiedlichen Teilchenfluss, eine unterschiedliche Teilchenenergie, eine unterschiedliche Teil-Strahl-Geometrie und dergleichen. Ein weiterer Vorteil bei der Berücksichtigung zumindest einer Bestrahlungs-Strahl-Inhomogenität besteht darüber hinaus darin, dass bei der Berechnung der Bestrahlungsplanung eine gewisse Mittelung zwischen unterschiedlichen Zielvolumenwerten, einschließlich des davorliegenden Materials, erfolgt. Während beispielsweise bei bislang üblichen Bestrahlungsplanungsverfahren bei einem Materialdichtewechsel (beispielsweise bei einem Übergang von einem Knochen in einen normalen Gewebebereich oder von einem normalen Gewebe zu einem luftgefüllten Hohlraum) ein sehr starker Sprung bei der Ansteuerung der Strahlerzeugungsvorrichtung erfolgt (was technologisch durchaus zu Problemen bei der Bestrahlungs-Strahl-Nachführung führen kann), stellt es üblicherweise einen sehr vorteilhaften "Nebeneffekt" dar, dass bei Anwendung des vorgeschlagenen Verfahrens üblicherweise eine eher langsamere, kontinuierliche Verstellung ausreicht. Dies kann nicht nur wirtschaftliche Vorteile mit sich bringen (beispielsweise weil die entsprechenden Nachsteuerungskomponenten kleiner dimensioniert werden können und/oder eine kurzzeitige Abschaltung des Bestrahlung-Strahls kürzer ausfallen kann oder sogar gänzlich entfallen kann), sondern kann auch zu einer Verbesserung der Güte der Bestrahlung beziehungsweise der Bestrahlungsplanung führen, da beispielsweise "Überschwinger" und ähnliche Effekte vermieden werden können. Mit dem vorliegend vorgeschlagenen Verfahren geht zwar in aller Regel eine zum Teil deutliche Erhöhung des numerischen Aufwands bei der Erstellung der Bestrahlungsplanung einher, jedoch kann hierdurch typischerweise eine zum Teil signifikante Verbesserung der Qualität der Bestrahlungsplanung und damit schlussendlich der Bestrahlung selbst erzielt werden. Darüber hinaus ist darauf hinzuweisen, dass der gegebenenfalls erforderliche zusätzliche numerische Aufwand mit heutigen schnellen Computern in der Regel beherrschbar ist.

Insbesondere kann es vorteilhaft sein, die Bestrahlungs-Strahl-Inhomogenität bei der Berechnung der Bestrahlungsplanungs-Datensätzen, insbesondere bei der Berechnung von "look-up-Tabellen (LUK) zu berücksichtigen, wenn der Bestrahlungsstrahl während der Bestrahlung nachgeführt wird. Insbesondere wenn die Bestrahlungs-Strahl-Inhomogenität bei der Berechnung von Kompensationsparameter berücksichtigt wird. Mitberücksichtigt kann hierbei generell werden, dass die Berechnung von Kompensationsparametern in der LUK-Tabelle die Inhomogenität des Bestrahlungs-Strahls als einen Eingabeparameter vorsieht.

Besonders vorteilhaft ist es, wenn zumindest eine Bestrahlungs-Strahl-Inhomogenität eine Inhomogenität bezüglich der Bestrahlungs-Strahlintensität, insbesondere hinsichtlich der ortsaufgelösten Bestrahlungs-Strahlintensität darstellt. So kann die Strahlintensität (beispielsweise bei einem Teilchenstrahl der Teilchenfluss pro Flächeneinheit und Zeiteinheit) zum Teil stark variieren. Dies kann einerseits eine zeitliche Variation und/oder eine örtliche Variation betreffen. So ist zum Beispiel der von einem Synchrotron bereitgestellte Teilchenstrahl zunächst einmal gepulst. Das heißt, dass nur in bestimmten Zeit-Intervallen ein Teilchenstrahl freigesetzt wird, während zu anderen Zeitpunkten kein Teilchenstrahl freigesetzt wird. Auch kann die freigesetzte Dosis während eines "Teilchenstrahl-Ausgabeintervalls" (ein so genannter Teilchen-Spill) variieren. So werden typischerweise am Anfang und am Ende eines Teilchen-Spills weniger Teilchen als "in der zeitlichen Mitte" des Teilchen-Spills freigesetzt. Weiterhin variiert in aller Regel der Teilchenfluss in Abhängigkeit von der relativen Lage im Teilchenstrahl. So ist der Teilchenfluss in der Mitte des Teilchenstrahls am höchsten, während er zu den Rändern des Teilchenstrahls hin abfällt (beispielsweise Gauß-förmig). Typischerweise hat ein Teilchenstrahl bei Hadronen-Teilchenstrahlen (insbesondere Schwerionen-Teilchenstrahlen) eine Halbwertsbreite von 3-10 mm. Erste Versuche haben ergeben, dass insbesondere die Berücksichtigung der genannten Inhomogenitäten des Bestrahlungs-Strahls zu üblicherweise besonders großen Verbesserungen der Qualität der Bestrahlungsplanung und/oder der Bestrahlung führen. Die rechnerische Berücksichtigung der Bestrahlungs-Strahl-Inhomogenitäten kann beispielsweise durch Verwendung eines unterschiedlichen Teilchenflusses in vorzugsweise geeignet geformten Teil-Bestrahlungs-Strahlen erfolgen. Auch ist es möglich, die Orts-Inhomogenität beispielsweise durch numerische Funktionen anzufitten (insbesondere durch eine Gauß-Verteilung), und hierdurch im Rahmen der Bestrahlungsplanung zu berücksichtigen. Während es bevorzugt ist, dass möglichst sämtliche Teile des Bestrahlungs-Strahls numerisch berücksichtigt werden, so kann es sich insbesondere zur Verringerung des numerischen Aufwands als vorteilhaft erweisen, dass nur Teilbereiche des Bestrahlungs-Strahls berücksichtigt werden. Insbesondere können in Vorab-Berechnungen Teilbereiche mit einem besonders starken Einfluss auf das Gesamtergebnis ermittelt werden, so dass die qualitative Verschlechterung der Ergebnisse relativ gering ausfallen kann. Die Berücksichtigung des gesamten Strahls und/oder lediglich von Teilen desselben kann übrigens zusätzlich oder alternativ auch im Zusammenhang mit anders gearteten Inhomogenitäten verwendet werden.

Besonders vorteilhaft ist es, wenn eine Berücksichtigung von Bewegungen zumindest eines Zielvolumenbereichs zumindest zeitweise und/oder zumindest bereichsweise unter Verwendung von Bewegungskompensation erfolgt, insbesondere unter Verwendung von Korrektur-Vektoren für die Ansteuerung der Strahlerzeugungsvorrichtung, insbesondere für die Ansteuerung des Bestrahlungs-Strahls. Mit der vorgeschlagenen Weiterbildung des Verfahrens ist es insbesondere möglich, zumindest teilweise und/oder zumindest zeitweise (in sich) bewegte Körper besonders genau und optimiert bestrahlen zu können. Insbesondere bietet sich die vorgeschlagene Weiterbildung des Verfahrens an, um so genannte Tracking-Verfahren realisieren zu können. Mit anderen Worten kann also insbesondere eine Bestrahlungs-Strahl-Inhomogenität bei der Erstellung von so genannten "look-up"-Tabellen im Rahmen der Erstellung einer Bestrahlungsplanung für "Tracking"-Verfahren berücksichtigt werden. Besondere Genauigkeitsgewinne können sich dabei insbesondere dadurch ergeben, dass Dehnungen, Stauchungen, "innere Relativverschiebungen" und "innere Verdrehungen" von Gewebe in Kombination mit der Bestrahlungs-Strahl-Inhomogenität mit berücksichtigt werden können. Insbesondere in einem Ring (einer Art "Halo") um das jeweils aktive Bestrahlungszentrum konnte es bei bislang verwendeten Verfahren oftmals zu stark variieren Dosiseinträgen (sowohl Unterdosierung, als auch Überdosierung) kommen. Aber auch der (an sich unerwünschte) Dosiseintrag in insbesondere proximal zum jeweils aktiven Bestrahlungszentrum liegende Gewebebereiche kann mit der vorgeschlagenen Weiterbildung besonders genau berechnet werden. Eine Bewegung kann dabei beispielsweise durch direkte Messung der Bewegung des Körpers (beispielsweise durch Computertomographie-Verfahren) und/oder unter Verwendung so genannter Bewegungssubstitute (beispielsweise Dehnungsmessstreifen, die um den Brustkorb eines Patienten gelegt werden, Bewegungsverfolgung mit Videosystemen und dergleichen) gemessen werden. Vorteilhaft ist es insbesondere, wenn sowohl direkte Messungen, als auch Bewegungssubstitute genutzt werden. Die bei einer derartigen "Parallelmessung" gewonnenen Bewegungssubstitute können insbesondere in Korrelation zu direkten Messungen gespeichert werden. Zu einem späteren Zeitpunkt (wie insbesondere bei der Applikation der Bestrahlungsplanung) können Bewegungssubstitut-Messungen besonders vorteilhaft genutzt werden, weil beispielsweise bei der Applikation der Bestrahlungsplanung oftmals keine direkten Bewegungs-Messungen erfolgen können. Durch die vorangegangene "Parallelmessung" kann jedoch mithilfe einer Bewegungssubstitut-Messung mit guter Genauigkeit auf den tatsächlichen Bewegungszustand geschlossen werden. Die Bewegungskompensation kann aber nicht nur in transversaler Richtung (im Wesentlichen senkrecht zum Bestrahlungs-Strahl), sondern insbesondere auch in longitudinaler Richtung (Variation der Eindringtiefe) des Bestrahlungsstrahls erfolgen. Es ist im Übrigen darauf hinzuweisen, dass bei Verwendung des vorgeschlagenen Verfahrens die für eine Bewegungskompensation erforderlichen Anpassungen pro Zeiteinheit oftmals geringer als bei bislang im Stand der Technik verwendeten Verfahren ausfallen. Die apparativen Anforderungen zur Realisierung einer Bewegungskompensation können daher meist geringer ausfallen, was insbesondere auch wirtschaftliche Vorteile mit sich bringen kann.

Vorteilhaft ist es weiterhin, wenn das Verfahren derart ausgeführt wird, dass sich die bei der Erstellung der Bestrahlungsplanung verwendete Ortsauflösung, insbesondere die Ortsauflösung des Bestrahlungs-Strahls, zumindest zeitweise und/oder zumindest bereichsweise an der Ortsauflösung einer Diagnoseeinrichtung, insbesondere einer bildgebenden Diagnoseeinrichtung orientiert. Erste Versuche haben ergeben, dass sich die Qualität der Bestrahlungsplanung beziehungsweise der Bestrahlung insbesondere dann steigern lässt, wenn eine derartige Anpassung erfolgt. Insbesondere numerische Artefakte (wie sie beispielsweise in anderen Bereichen der Technik als Moire-Effekt bekannt sind) können dadurch oftmals verringert oder vermieden werden. Insbesondere hat sich ergeben, dass sich mit einer präziseren Berücksichtigung des Bestrahlungs-Strahls im Vergleich zur Auflösung einer Diagnoseeinrichtung in aller Regel keine weitere Verbesserung der Bestrahlungsplanung/der Bestrahlung erzielen lässt. Hierdurch kann insbesondere ein unnötiges Anwachsen des numerischen Rechenaufwands vermieden werden. Auch kann es sich als sinnvoll erweisen, wenn beispielsweise für die Bestrahlungsplanung ein Teil-Bestrahlungs-Strahl pro zwei oder pro vier (=zwei mal zwei) Pixeln einer bildgebenden Diagnoseeinrichtung erfolgt. Hierdurch kann der numerische Aufwand nochmals verringert werden und insbesondere können numerische Artefakte vermieden werden. Auch an 1, 2, 3, 4, 5, 6, 7, 8, 9 und/oder 10 mal 1, 2, 3, 4, 5, 6, 7, 8, 9 und/oder 10 große "Überraster" kann selbstverständlich gedacht werden. Die Ortsauflösung der Diagnoseeinrichtung kann sich dabei insbesondere an den aktuellen Rahmenparametern orientieren. Beispielsweise können die Daten der Diagnoseeinrichtung in Form von Volumenbereichen (so genannte "Voxel") vorliegen. Die Ortsauflösung der Diagnoseeinrichtung kann dann durch einen Flächenschnitt durch das dreidimensionale Voxelgitter erfolgen, wobei die Schnittebene insbesondere (im Wesentlichen) senkrecht zum einfallenden Bestrahlungs-Strahl steht. Möglich ist es sowohl, eine möglichst typische Standard-Schnittfläche zu wählen (so dass es beispielsweise beim Durchrechnen einer Gesamt-Bestrahlungsplanung zu gewissen Winkeltoleranzen kommen kann), aber auch, die Schnittfläche jeweils "passend" zum aktuellen (Teil-)Bestrahlungs-Strahl zu wählen. Grundsätzlich kann es sich bei der Diagnoseeinrichtung um eine beliebige Art von Diagnoseeinrichtung handeln. Auch Kombinationen von unterschiedlichen Messverfahren sind selbstverständlich denkbar. Bevorzugt sind dabei bildgebende Diagnoseeinrichtungen (speziell dreidimensionale beziehungsweise vierdimensionale bildgebende Diagnoseeinrichtungen) wie beispielsweise Röntgenbilder, Computertomogramme, Kernspin-Tomogramme und dergleichen.

Der Bestrahlungs-Strahl ist ein Bestrahlungs-Strahl ein Hadronen-Strahl, besonders bevorzugt ein Protonen-Strahl und/oder ein Schwerionen-Strahl. Gerade die genannten Teilchensorten weisen einen besonders ausgeprägten Bragg-Peak auf, so dass ein Dosiseintrag auch dreidimensional gut strukturiert werden kann. Möglich ist es aber auch, anderweitige Hadronen, wie beispielsweise Pionen, Mesonen und dergleichen zu verwenden. Unter Schwerionen sind insbesondere sämtliche Teilchensorten (beziehungsweise deren Ionen) zu verstehen, die eine Masse aufweisen, die größer als die von Protonen ist. Insbesondere ist an Helium zu denken (insbesondere Helium 3 und Helium 4) sowie Neon, Sauerstoff, Kohlenstoff, Silizium und dergleichen.

Bei dem Verfahren die Bestrahlung zumindest zeitweise und/oder zumindest teilweise durch ein Raster-Scan-Verfahren, durch ein Spot-Scan-Verfahren und/oder durch ein kontinuierliches Scan-Verfahren erfolgt. Mit derartigen Verfahren ist ein besonders genauer und fein dosierbarer Dosiseintrag möglich. Darüber hinaus ist mit derartigen Verfahren auch eine Bewegungskompensation besonders vorteilhaft und besonders genau realisierbar. Bei einem Raster-Scan-Verfahren wird der Bestrahlungs-Strahl sukzessive an die verschiedenen Punkte des Bestrahlungsgitters bewegt. Je nach Dosis, die am entsprechenden Gitterpunkt zu deponieren ist, verbleibt der Strahl kürzer oder länger auf dem Gitterpunkt. Zwischen zwei Gitterpunkten wird der Bestrahlungs-Strahl dabei nicht abgeschaltet. Bei einem Spot-Scan-Verfahren erfolgt die Bestrahlung ähnlich zu einem Raster-Scan-Verfahren. Als Unterschied wird dieser jedoch beim Wechsel zwischen zwei Gitterpunkten kurzzeitig ausgeschaltet. Bei einem kontinuierlichen Scan-Verfahren erfolgt die Bewegung des Strahls kontinuierlich. Je nach der zu deponierenden Dosis ist die Bestrahlungs-Strahl-Bewegung schneller oder langsamer. Scan-Verfahren sind besonders effektiv in Kombination mit einem möglichst feinen Teilchenstrahl. Ein solcher feiner Teilchenstrahl wird oftmals auch als bleistiftdünner Teilchenstrahl oder fachsprachlich auch als "pencil beam" bezeichnet.

Weiterhin erweist es sich als vorteilhaft, wenn bei dem Verfahren zumindest zeitweise und/oder zumindest bereichsweise eine unterschiedliche Gewichtung unterschiedlicher Bereiche des Bestrahlungs-Strahls erfolgt. Die unterschiedliche Gewichtung kann in an sich beliebiger Weise erfolgen. Insbesondere ist es möglich, dass die Gewichtung beispielsweise basierend auf der (lokalen) Bestrahlungs-Strahlintensität des Bestrahlungs-Strahls beziehungsweise des dazu korrespondierenden Teil-Bestrahlungs-Strahls erfolgt. In jedem Fall können durch die Gewichtung bestimmte physikalische, gegebenenfalls auch biologische und sonstige Effekte in die Bestrahlungsplanung einfließen, so dass schlussendlich eine bessere Bestrahlungsplanung beziehungsweise eine bessere Bestrahlung resultieren kann.

Möglich ist es dabei insbesondere, dass Überlappungsbereiche bei der Bestrahlung benachbarter Bestrahlungspunkte mit dem Bestrahlungs-Strahl zumindest zeitweise und/oder zumindest bereichsweise gewichtet, insbesondere stärker gewichtet, berücksichtigt werden. Da in dem Überschneidungsbereich in Summe eine vergleichsweise hohe Dosis deponiert wird (im Vergleich zu sonstigen Punkten im Bestrahlungsbereich des Bestrahlungs-Strahls) können genauere Ergebnisse erzielt werden, wenn die entsprechenden Überlappungsbereiche hierzu korrespondierend stärker gewichtet werden. Auch die "Nebeneffekte" (wie beispielsweise eine langsamere Veränderung von Bestrahlungs-Strahl-Parametern) können hierdurch gegebenenfalls verbessert werden. In diesem Zusammenhang ist darauf hinzuweisen, dass benachbarte Bestrahlungspunkte (beispielsweise Rasterpunkte bei einem Scan-Verfahren) bei heutigen Strahlerzeugungsvorrichtungen typischerweise etwa 33% der Strahlhalbwertsbreite voneinander entfernt liegen. Mit anderen Worten gibt es einen relativ großen Überlapp zwischen zwei benachbarten Bestrahlungs-Punkten.

Eine weitere bevorzugte Weiterbildung des Verfahrens ergibt sich, wenn zumindest zeitweise und/oder zumindest bereichsweise eine Gewichtung basierend auf Materialparametern erfolgt, insbesondere basierend auf ortsveränderlichen Materialparametern. Eine derartige Weiterbildung ist insbesondere dann von Vorteil, wenn die entsprechenden Materialparameter relativ starke Schwankungen aufweisen, insbesondere relativ starke Schwankungen in einem relativ kleinen Variationsbereich, wie beispielsweise über eine relativ kurze Strecke hinweg. Durch eine entsprechend geeignete Gewichtung kann dadurch die starke Schwankung des Materialparameters in einem gewissen Sinne "ausgemittelt" werden. Dadurch kann die Qualität der Bestrahlung gegebenenfalls verbessert werden. Auch ist es möglich, dass starke Änderungen bestimmter Parameter der Strahlerzeugungsvorrichtung, insbesondere starke Schwankungen der Ansteuerparameter des Bestrahlungs-Strahls zumindest in einem gewissen Umfang vermieden werden können. Unter Materialparametern können im Falle von biologischen Körpern insbesondere auch physiologische Parameter zu verstehen sein. Rein beispielhaft kann hierbei der Wassergehalt, die Dichte, der Kohlenstoffgehalt, das Wasseräquivalent, die Elektronendichteverteilung, die wasseräquivalente Eindringtiefe und dergleichen verwendet werden. Eine Verbesserung der Bestrahlung beziehungsweise der Bestrahlungsplanung kann sich insbesondere bei Übergängen von Knochen zu normalen Gewebe und/oder bei Übergängen von normalem Gewebe zu luftgefüllten Körperbereichen ergeben.

Eine weitere vorteilhafte Weiterbildung des Verfahrens ergibt sich, wenn die Wegführung des Scan-Pfads insbesondere im Rahmen der Bestrahlungsplanung zumindest zeitweise und/oder zumindest bereichsweise berücksichtigt wird, insbesondere verändert wird, besonders bevorzugt optimiert wird. Hier ist es möglich, beispielsweise starke Materialparameterschwankungen von vornherein zu berücksichtigen, so dass sich die Qualität der Bestrahlungsplanung beziehungsweise der Bestrahlung insgesamt verbessern kann.

Insbesondere ist es in diesem Zusammenhang möglich, dass die Wegführung des Scan-Pfads zumindest zeitweise und/oder zumindest bereichsweise im Rahmen der Erstellung der Bestrahlungsplanung in die Berechnung der "look-up"-Tabellen einfließt und gegebenenfalls auch eine Rückkopplung auf die Wegführung des Scan-Pfads erfolgt. Besonders bevorzugt ist es, wenn der Strahl so verändert, insbesondere optimiert wird, dass starke "Kontrastübergänge" zumindest zum Teil, vorzugsweise möglichst weitgehend und/oder im Wesentlichen vollständig vermieden werden können. Rein beispielhaft kann bei einem biologischen Körper eine Führung des Scanpfads parallel zu einer Rippe (anstatt senkrecht hierzu) erfolgen. In einem solchen Fall sind die durch die Änderung der Gewebedichte erforderlichen Tiefenmodulations-Sprünge (die beispielsweise eine Verstellung einer passiven Modulatoreinrichtung erforderlich machen) weitgehend reduziert. Hierdurch kann die Strahlerzeugungsvorrichtung einfacher gestaltet werden und/oder die Bestrahlung beziehungsweise die Bestrahlungsplanung verbessert werden. Eine derartige Veränderung beziehungsweise Optimierung des Scanpfads kann beispielsweise unter Verwendung von Kosten-Funktionen oder dergleichen realisiert werden. Hierzu können insbesondere auch Daten herangezogen werden, die im Rahmen der Gewichtung unterschiedlicher Bestrahlungspunkte gewonnen werden, beziehungsweise können vorab gewonnene Daten für eine derartige Gewichtung herangezogen werden.

Die erstellten Bestrahlungsplanungen und die anschließende Behandlung kann sowohl für Mensch und Tier, aber auch für so genannten Phantome ("Patienten-Dummys") oder auch für allgemeine Körper (beispielsweise in den Materialwissenschaften) berechnet werden, beziehungsweise an diesen durchgeführt werden.

Weiterhin wird ein Verfahren zur Ansteuerung einer Strahlerzeugungsvorrichtung zur Applizierung einer ortsaufgelösten Strahlendosis in zumindest einem zumindest zeitweise und/oder zumindest bereichsweise bewegten Zielvolumenbereich unter Verwendung der Strahlerzeugungsvorrichtung vorgeschlagen, bei dem zumindest bereichsweise eine Bestrahlungsplanung verwendet wird, welche nach einem Verfahren vom vorab beschriebenen Typ erstellt wurde. Auf diese Weise können die bereits vorab beschriebenen Vorteile und Eigenschaften auch für die eigentliche Bestrahlung eines Körpers genutzt werden. Bei dem Körper kann es sich in beliebiger Weise um einen technischen Körper (beispielsweise im Rahmen der Materialwissenschaften), um ein Patienten-Phantom aber auch um einen Patienten selbst handeln. Die bereits vorab beschriebenen Vorteile und Eigenschaften ergeben sich dann auch für die eigentliche Bestrahlung in analoger Weise.

Weiterhin wird eine Vorrichtung zur Erstellung einer Bestrahlungsplanung vorgeschlagen, welche derart ausgebildet und eingerichtet ist, dass diese zumindest bereichsweise ein Verfahren mit den vorab genannten Eigenschaften durchführt. Mit der Vorrichtung lassen sich dann die bereits genannten Vorteile und Eigenschaften in analoger Weise erzielen. Auch ist es möglich, die Vorrichtung im Sinne der obigen Beschreibung zu modifizieren beziehungsweise weiterzubilden. Die Vorrichtung selbst kann grundsätzlich ein entsprechend programmtechnisch eingerichteter elektronischer Rechner, wie beispielsweise ein Personalcomputer oder eine Workstation sein. Auch eine Zusammenschaltung mehrerer PCs beziehungsweise Workstations (so genannter Rechner-Cluster oder Rechnerverbund) ist selbstverständlich denkbar. Auch können die Vorteile und Eigenschaften aus verteilten Netzwerken (fachsprachlich oftmals als distributed computing bezeichnet) genutzt werden. Im Übrigen soll es auch möglich sein, ein Computerprogrammprodukt als solches beanspruchen zu können, welches in Kombination mit einem Computer eine derartige Vorrichtung ergibt.

Weiterhin wird eine Vorrichtung zur Ansteuerung zumindest einer Strahlerzeugungsvorrichtung und/oder eine Strahlerzeugungsvorrichtung vorgeschlagen, welche zumindest eine Vorrichtung aufweist, die derart ausgebildet und eingerichtet ist, dass diese zumindest teilweise ein Verfahren vom vorab beschriebenen Typ durchführt. Auch die Vorrichtung zur Ansteuerung zumindest einer Strahlerzeugungsvorrichtung beziehungsweise die Strahlerzeugungsvorrichtung selbst weist dann die bereits vorab beschriebenen Eigenschaften und Vorteile in analoger Weise auf. Auch diese Vorrichtung beziehungsweise Strahlerzeugungsvorrichtung kann im Sinne der vorherigen Beschreibung modifiziert werden. Bei der Strahlerzeugungsvorrichtung handelt es sich um einen Teilchenbeschleuniger, insbesondere für Hadronen (Protonen und/oder Schwerionen). Hierbei können zumindest teilweise Linearbeschleuniger, Synchrotrons und dergleichen Beschleuniger eingesetzt werden.

Im Folgenden wird die Erfindung anhand vorteilhafter Ausführungsbeispiele und unter Bezugnahme auf die beigefügte Zeichnung näher erläutert. Es zeigen:
- Fig. 1:: eine Vorrichtung zur Durchführung einer Bestrahlung in einer schematischen Perspektivansicht;
- Fig. 2:: die Struktur eines Teilchenstrahls in einem schematischen Querschnitt;
- Fig. 3:: eine schematische Veranschaulichung unterschiedlicher Scan-Pfade eines Teilchenstrahls;
- Fig. 4:: ein Verfahren zur Erstellung einer Bestrahlungsplanung in einem schematischen Ablaufplan.

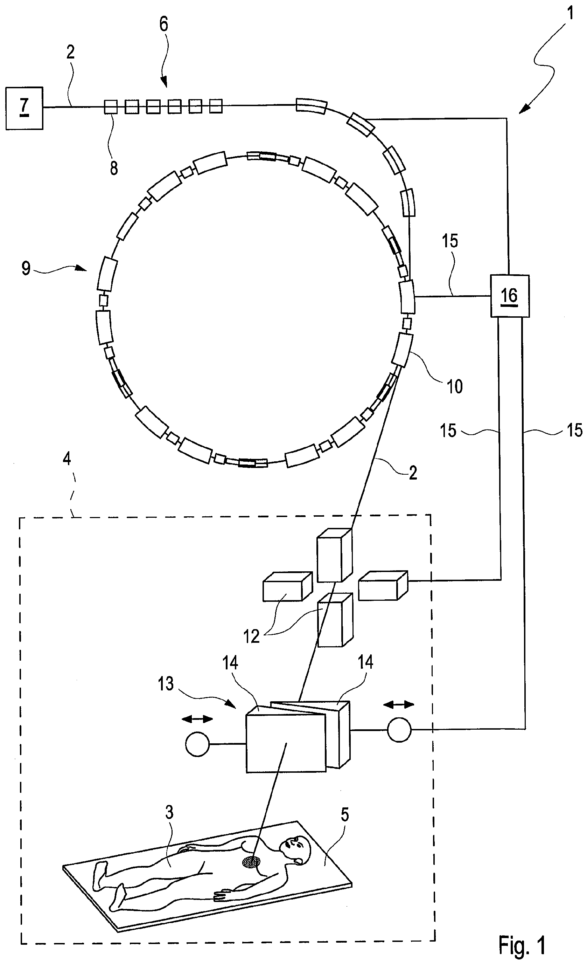

In Fig. 1 ist in einer schematischen, perspektivischen Darstellung eine Bestrahlungsanlage 1 dargestellt, mit der eine vorab erstellte Bestrahlungsplanung in einen mit dem Teilchenstrahl 2 zu beaufschlagenden Körper 3 appliziert wird. Bei dem Körper 3 kann es sich grundsätzlich um einen beliebigen Materialblock handeln. Im vorliegend dargestellten Ausführungsbeispiel handelt es sich bei dem zu bestrahlenden Körper 3 um einen Patienten 3, der sich in einem Behandlungsraum 4 auf einer Liege 5 befindet und vorzugsweise auf dieser fixiert ist. Ebenso ist es jedoch auch denkbar, dass anstatt eines Patienten 3 ein so genannter Patienten-Dummy oder ein sonstiges Phantom von der Bestrahlungsanlage 1 bestrahlt wird.

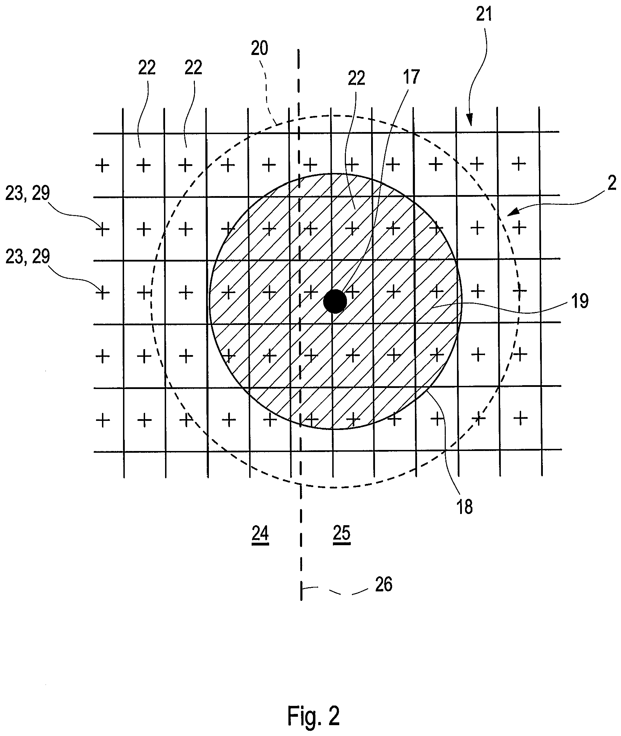

Die Bestrahlungsanlage 1 ist als an sich bekannter Schwerionenbeschleuniger 6 ausgebildet. Der Schwerionenbeschleuniger 6 weist eine Ionenquelle 7 auf. Die von der Ionenquelle 7 erzeugten Ionen (die als Teilchenstrahl 2 vorliegen) werden zunächst in einem Linearbeschleuniger 8 vorbeschleunigt und in einen Synchrotronring 9 eingeschossen. Im Synchrotronring 9 werden die Ionen weiter auf die erwünschte, hohe Zielenergie beschleunigt. Ist die gewünschte Zielenergie erreicht, werden die im Synchrotronring 9 befindlichen Teilchen über ein Extraktionsseptum 10 extrahiert. Eine typische Zeitdauer für einen Extraktionszyklus (ein so genannter Teilchenspill) liegt zwischen 2 und 10 s. Die durch das Extraktionsseptum 10 extrahierten Ionen werden dem Patienten 3, der sich im Behandlungsraum 4 befindet, zugeführt. Die Applikation der Teilchen des Teilchenstrahls 2 erfolgt im vorliegend dargestellten Ausführungsbeispiel unter Verwendung eines Scanvorgangs, speziell eines so genannten Raster-Scanvorgangs. Hierzu wird der bleistiftdünne Teilchenstrahl 2 derart zeilen-, spalten- und scheibenweise geführt, dass ein erwünschtes dreidimensionales Volumen mit einer erwünschten Solldosis abgerastert wird und somit das Volumen bestrahlt wird (wobei gewisse Schwankungen bei der tatsächlich applizierten Dosis nie ganz zu vermeiden sind). Je nachdem, wie hoch die im jeweiligen Rasterpunkt 29 (vergleiche Fig. 2) einzubringende Einzeldosis ist, verharrt der Teilchenstrahl 2 beim Raster-Scanvorgang unterschiedlich lang in der jeweiligen Rasterpunkt-Position 29.

Der Teilchenstrahl 2 wird in einer lateralen Richtung durch ein Ablenkspulenpaar 12 abgelenkt und somit seine Position im Volumen gezielt verändert.

In longitudinaler Richtung (Veränderung der Lage des Bragg-Peaks der Ionen beziehungsweise Eindringtiefe der Ionen) erfolgt eine Veränderung durch geeignete Ansteuerung eines passiven Energiemodulators 13. Bei diesem werden in an sich bekannter Weise zwei keilförmige Blöcke 14 aus energieabsorbierendem Material derart bewegt, insbesondere verfahren, dass der Teilchenstrahl 2 für unterschiedliche Positionen der Blöcke 14 eine unterschiedliche Strecke durch das energieabsorbierende Material der Blöcke 14 zurücklegen muss. Dadurch werden die durch den passiven Energiemodulator 13 hindurchtretenden Ionen unterschiedlich stark energetisch abgebremst und die Energie des Teilchenstrahls kann somit moduliert werden. Selbstverständlich ist auch eine andere Bauweise des passiven Energiemodulators 13 möglich, beispielsweise mit einer abweichenden Anzahl von keilförmigen Blöcken 14. Auch sind andere Formen von Energiemodulatoren denkbar.

Die einzelnen Komponenten der Bestrahlungsanlage 1 stehen über Datenleitungen 15 mit einer Steuereinheit 16 in Verbindung. Die Steuereinheit 16 kann aus einem einzelnen Rechner oder einer größeren Anzahl an Rechnern bestehen. Als Rechner sind nicht nur klassische Computer denkbar, sondern insbesondere auch (teilweise) Einplatinen-Computer und dergleichen. Insbesondere können in der Steuereinheit 16 vor der eigentlichen Applizierung der Solldosisverteilung die Daten der vorab errechneten Bestrahlungsplanung 36 geladen werden und anschließend die entsprechenden Komponenten der Bestrahlungsanlage 1 geeignet angesteuert werden. Dazu kann die Steuereinheit 16 für die jeweiligen Komponenten 7, 8, 9, 10, 12, 13 Steuersignale erzeugen und über die Datenleitungen 15 an diese weiterleiten. Darüber hinaus werden von der Steuereinheit 16 Mess- und Kontrollsignale, die von geeigneten Sensoren beziehungsweise Messgeräten erfasst werden (in Fig. 1 nicht dargestellt), empfangen und geeignet verarbeitet. Im Übrigen ist es auch möglich, dass die eigentliche Bestrahlungsplanung (beziehungsweise eine nachgeschaltete Optimierung derselben) durch die Steuereinheit 16 errechnet wird. Bevorzugt ist es jedoch, wenn die Bestrahlungsplanung auf einer separaten Vorrichtung errechnet wird und die Steuereinheit 16 lediglich zur Applizierung der Bestrahlungsplanung und gegebenenfalls zu einer gegebenenfalls kurz vor der Applizierung erfolgenden Nachoptimierung der Bestrahlungsplanung verwendet wird. Bei Verwendung einer Nachoptimierung ist es insbesondere möglich, dass aktuelle, insbesondere tagesaktuelle Daten über die Beschleunigungsanlage 1 (beispielsweise Form und Intensitätsverteilung des Teilchenstrahls 2, Intensitätsverteilung eines Teilchenspills und dergleichen) und/oder des Patienten (beispielsweise kleinere Größenveränderungen oder Lageveränderungen des zu bestrahlenden Tumors) berücksichtigt werden können.

In Fig. 2 ist in einer schematischen Querschnittsansicht die "Struktur" des Teilchenstrahls 2 dargestellt. Der in der Mitte zu erkennende Punkt stellt den Mittelpunkt 17 des Teilchenstrahls 2 dar. Der Teilchenstrahl 2 weist jedoch eine endliche Ausdehnung auf. Dabei weist der Teilchenstrahl 2 keine homogene Intensität mit einer scharfen Abbruchkante auf. Vielmehr verläuft die Intensitätsverteilung des Teilchenstrahls 2 nach Art einer Gaußkurve. In Fig. 2 ist daher neben den Mittelpunkt 17 des Teilchenstrahls 2 die 50%-Linie 18 dargestellt, an der die Maximalintensität des Teilchenstrahls 2 (die beim vorliegend dargestellten Beispiel mit dem Mittelpunkt 17 des Teilchenstrahls 2 übereinstimmt) auf 50% abgesunken ist. Der schraffierte Bereich 19 stellt somit den "Hauptteil" des Teilchenstrahls 2 dar. Weiterhin ist in Fig. 2 die 10%-Linie 20 eingezeichnet, bei der die Maximalintensität des Teilchenstrahls 2 auf 10% abgefallen ist. Außerhalb des Bereichs der 10%-Linie 20 erfolgt daher ein nur noch geringer Dosiseintrag. Ein typischer Wert für die Größe des Teilchenstrahls 2 beträgt etwa 3-10 mm Durchmesser an der 50%-Linie 18. Der Vollständigkeit halber sollte darauf hingewiesen werden, dass der Teilchenstrahl 2 auch eine andere Intensitätsverteilung aufweisen kann. Darüber hinaus ist es möglich, dass der Teilchenstrahl 2 nicht rotationssymmetrisch ausgebildet ist. Beispielsweise können die 50%-Linie 18 beziehungsweise die 10%-Linie 20 auch eine Art elliptische Form beziehungsweise eine eiartige Form aufweisen.

Zusätzlich ist in Fig. 2 das Gitter 21 zu erkennen, welches die Auflösung eines Computertomogramms darstellt. Wie man erkennt, ist die Auflösung des Computertomogramms höher als die des Teilchenstrahls 2 (insbesondere der 50%-Linie 18 des Teilchenstrahls 2). Dadurch liegen mehrere Pixel 22 (also einzelne Zellen des Gitters 21) des Computertomogramms innerhalb der Strahlausdehnung und insbesondere innerhalb des schraffierten Bereichs 19.

Bei bisherigen Verfahren zur Berechnung von Bestrahlungsplanungen wird in der Regel lediglich der Mittelpunkt 17 des Teilchenstrahls 2 als Referenz für die Strahlposition des Teilchenstrahls 2 im Gitter 21 des Computertomogramms verwendet. Es ist leicht einsichtig, dass es insbesondere dann zu Fehlern kommen kann, wenn unmittelbar benachbart zum Mittelpunkt 17 des Teilchenstrahls 2 eine starke Diskontinuität 26 auftritt. Eine derartige Diskontinuität 26 kann beispielsweise im Brustbereich längs einer einzelnen Rippe 24 vorliegen. Wird der Teilchenstrahl 2 hier senkrecht zur Begrenzungskante der Rippe 24 bewegt, so erfolgt eine starke Schwankung beim Übergang vom Knochenbereich zum normalen Gewebebereich 25. Dies hat zur Folge, dass bei der Bestrahlungsplanung eine diskontinuierliche Änderung der Eindringtiefe der Teilchen des Teilchenstrahls 2 vorliegt und dementsprechend die Energie des Teilchenstrahls 2 durch eine entsprechende Ansteuerung des passiven Energiemodulators 13 geändert werden muss. Darüber hinaus ist einsichtig, dass es beispielsweise im von der 50%-Linie 18 abgedeckten Rippenbereich 24 zu nicht unerheblichen Fehldosierungen kommen kann (insbesondere wenn der Mittelpunkt 17 des Teilchenstrahls 2 "gerade eben" im normalen Gewebebereich 25 liegt).

Vorliegend wird daher vorgeschlagen, dass gemäß einem Ausführungsbeispiel für ein Verfahren zur Erstellung einer Bestrahlungsplanung für jedes Pixel 22 der jeweils individuelle Beitrag des entsprechenden Teils des Teilchenstrahls 2 bei der Erstellung der Bestrahlungsplanung berücksichtigt wird. Bei der Erstellung der Bestrahlungsplanung werden somit mit anderen Worten im vorliegend dargestellten Beispiel mehrere "Teil-Teilchenstrahlen" verwendet. Die jeweilige Intensität eines Teil-Teilchenstrahls (entsprechend einer Pixelgröße 22) kann dabei beispielsweise durch analytische Verfahren sehr genau bestimmt werden. Möglich ist es aber auch, dass zur Verringerung des numerischen Aufwands angenäherte Werte in einer Look-Up-Table abgespeichert sind. Auch ist es möglich, dass zur Vereinfachung jeweils der Zentralpunkt 23 eines einzelnen Pixels 22 zu Zwecken der Erstellung der Bestrahlungsplanung verwendet wird. Im Übrigen ist es möglich (und in der Regel sinnvoll), dass die einzelnen Zentralpunkte 23 gleichzeitig die Bestrahlungspositionen (Rasterpunkt 29) bei der Durchführung eines Raster-Scanvorgangs darstellen. Möglich ist es aber auch, dass beispielsweise nur für jeweils 2, 4, 6, 9 usw. Pixel 29 eine einzelne Rasterposition 23 berechnet wird.

Um den numerischen Aufwand für die Erstellung der Bestrahlungsplanung weiter zu vereinfachen ist es darüber hinaus möglich, dass nur Teilchenstrahlbereiche, welche sich in einem gewissen Maximalabstand zum Mittelpunkt 17 des Teilchenstrahls 2 befinden, bei der Erstellung der Bestrahlungsplanung berücksichtigt werden. So ist es beispielsweise möglich, dass Pixel 22, welche (vollständig) außerhalb der 50%-Linie 18 oder der 10%-Linie 20 liegen, nicht in die Berechnung der Bestrahlungsplanung einfließen. Selbstverständlich sind auch andere Untereinheiten von Pixeln 22 denkbar. Diese werden im Allgemeinen als Subsets bezeichnet.

Durch die Berücksichtigung einer größeren Anzahl von Pixeln 22 beziehungsweise von Teil-Teilchenstrahlen kann nicht nur die Genauigkeit der Bestrahlungsplanung und schlussendlich der Bestrahlung erhöht werden. Darüber hinaus kann es dadurch zu einem "Verschmieren" von Diskontinuitäten 26 kommen, so dass die Anforderungen an die Verstellgeschwindigkeit des Ablenkspulenpaars 12 und insbesondere des Energiemodulators 13 deutlich geringer ausfallen können.

Im Übrigen wird bereits aus Fig. 2 ersichtlich, dass die Teilchenstrahlbereiche 19, 20 bei Bestrahlung jeweils zwei zueinander benachbarter Rasterpunkte 23 jeweils stark miteinander überlappen können. Hier ist es möglich, dass diese Überlappungsbereiche 27 im Zuge der Berechnung der Bestrahlungsplanung zusätzlich stärker gewichtet werden (also über die "einfache" Berücksichtigung der jeweiligen Strahlintensität in diesem Bereich hinaus). Typischerweise sind benachbarte Rasterpunkte 29 ein Drittel der Strahlhalbwertsbreite 18 des Teilchenstrahls 2 voneinander entfernt. Der dadurch resultierende Überlapp 27 zwischen zwei benachbarten Rasterpunkten 29 ist dementsprechend groß.

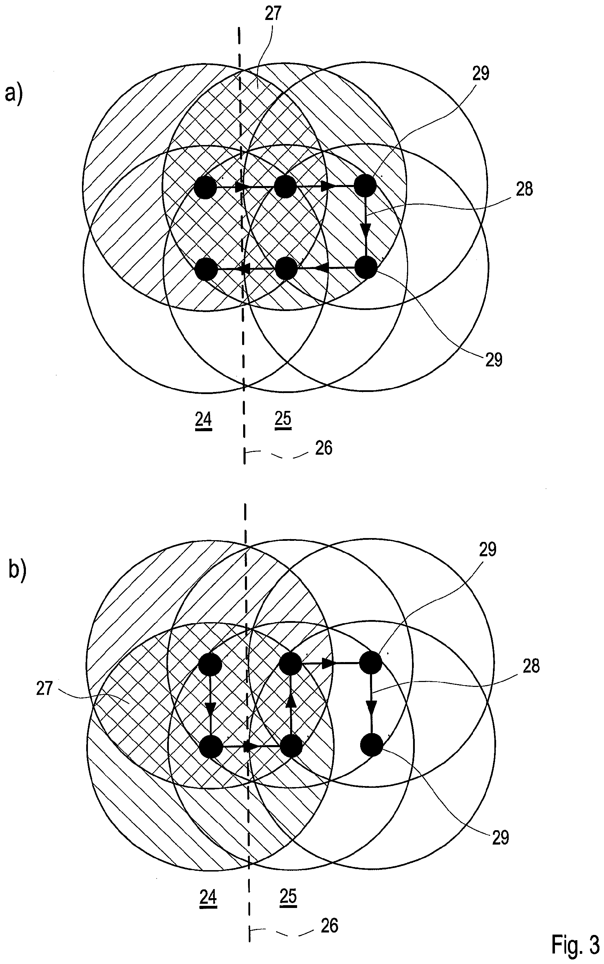

Dieses Überlappen 27 der Teilchenstrahlbereiche bei Bestrahlung zwei zueinander benachbarter Rasterpositionen 29 ist gut in Fig. 3 zu erkennen. Weiterhin wird aus einem Vergleich der Teilbilder a) und b) von Fig. 3 deutlich, wie sich eine stärkere Gewichtung des Überlappungsbereichs 27 in Verbindung mit einer entsprechenden Variation des Scanpfads 28 positiv auf die Qualität der Bestrahlung auswirken kann.

Erneut wird - analog zu Fig. 2 - von einer Gewebediskontinuität 26 ausgegangen, die sich beispielsweise im Brustkorbbereich bei einem Übergang von einem Rippenbereich 24 zu einem "Normalgewebe"-Bereich 25 ergibt. Beim ursprünglichen Scanpfad 28 von Fig. 3a wird die Diskontinuität 26 mehrfach überschritten. Dementsprechend häufig muss die Energiemodulatoreinheit 13 die Energie des Teilchenstrahls 2 innerhalb relativ kurzer Zeit über einen relativ großen Bereich hinweg anpassen. Dies setzt einen entsprechend schnellen Energiemodulator 13 voraus. Gegebenenfalls muss sogar der Teilchenstrahl 2 kurzzeitig abgeschaltet werden, um die erforderliche Energiemodulation realisieren zu können bevor der Teilchenstrahl 2 mit nunmehr passender Energie erneut eingeschaltet werden kann. Wird nun der Überlappungsbereich 27 entsprechend stark gewichtet und werden hierbei insbesondere auch Reichweitenunterschiede von im Überlappungsbereich 27 befindlichen Pixeln 22 (Pixelgitter 21 in Fig. 3 aus Übersichtlichkeitsgründen nicht eingezeichnet) entsprechend stark gewichtet, so kann diese Diskontinuität 26 beispielsweise im Rahmen einer Kostenfunktion erkannt werden. Liefert die Kostenfunktion ein entsprechend "negatives" Ergebnis, so wird versuchsweise ein anderer Scanpfad 28 verwendet.

Nach mehreren Iterationen bei der Erstellung der Bestrahlungsplanung kann sich beispielsweise der in Fig. 3b eingezeichnete Scanpfad 28 ergeben. Es wird deutlich, dass hier die Diskontinuität 26 nur ein einziges Mal vom Scanpfad 28 geschnitten wird. Die damit einhergehenden negativen Effekte fallen dementsprechend geringer aus. Wie man aus Fig. 3b ohne weiteres qualitativ erkennen kann, ändert sich die "Tiefenverteilung" der einzelnen Pixel 22 nicht beziehungsweise nur geringfügig, wenn der Teilchenstrahl 2 von einem Rasterpunkt 29 zum nächsten bewegt wird. Dementsprechend "positiv" ist dann auch das Verhalten der Kostenfunktion.

Lediglich der Vollständigkeit halber wird darauf hingewiesen, dass in Fig. 3a, 3b aus Anschauungsgründen lediglich ein kleiner Ausschnitt des Gitters 21 aus Rasterpunkten 29 dargestellt ist. In der Realität weist ein Bestrahlungsbereich ein Gitter 21 mit einer Gittergröße von mehreren 100 mal mehreren 100 bis hin zu mehreren 1000 mal mehreren 1000 Rasterpunkten 29 auf.

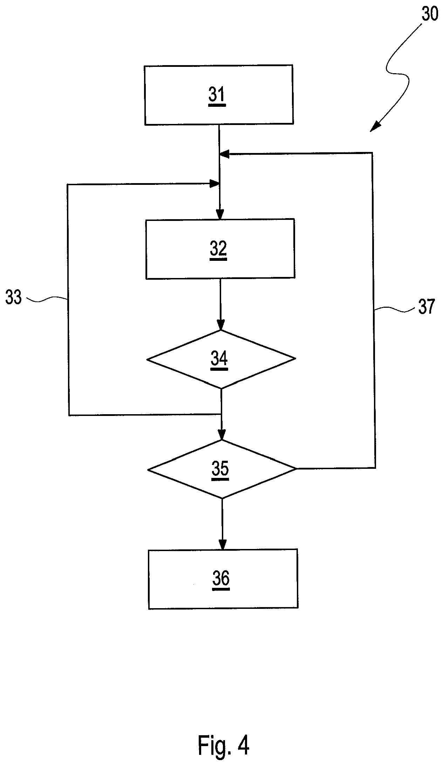

In Fig. 4 ist nochmals in einem schematischen Ablaufplan ein Verfahren 30 zur Erstellung einer Bestrahlungsplanung dargestellt. Zunächst werden in einem ersten Schritt 31 die für die Erstellung der Bestrahlungsplanung erforderlichen Daten eingelesen 31. Hierbei kann es sich insbesondere um durch Diagnoseverfahren ermittelte Erkenntnisse über Lage, Art, Größe und Verteilung eines Tumorgewebes in einem Patienten handeln. Hier sind insbesondere bildgebende Verfahren vorteilhaft, wie beispielsweise Computertomographieverfahren oder Kernspintomographieverfahren. Es kann sich aber auch um Parameter handeln, welche Art, Aufbau und Charakteristika einer Bestrahlungsanlage 1 beschreiben. Insbesondere wird im ersten Schritt 31 auch eine vom Arzt verschriebene Soll-Dosisverteilung eingelesen, die die Basis für die anschließende Bestrahlungsplanung darstellt.

Anschließend wird in einer Programmschleife 33 für jede einzelne Rasterposition 29 der jeweilige Strahlungseintrag des Teilchenstrahls 2 auf das Gewebe des Patienten 2 (oder eines anderen Materialkörpers beziehungsweise insbesondere auch eines Patienten-Dummys) berechnet 32. Insbesondere erfolgt ein Dosiseintrag nicht nur in das eigentlich erwünschte Zielvoxel, sondern auch in benachbart liegende Voxel und insbesondere in strahlaufwärts zum Zielvoxel liegende Gewebebereiche. Die Schleife 33 wird so lange durchlaufen, bis in einem Überprüfungsschritt 34 festgestellt wurde, dass das gesamte Gitter durchlaufen wurde. Anschließend wird in einem zweiten Überprüfungsschritt 35 überprüft, ob das Verfahren 30 bereits zu einem (lokalen) Optimum der resultierenden Dosisverteilung geführt hat (mit anderen Worten, ob die berechnete Dosisverteilung möglichst gut mit der vom Arzt verschriebenen Soll-Dosisverteilung übereinstimmt). Ist dies der Fall, endet das Verfahren 30 und die gewonnene Bestrahlungsplanung wird ausgegeben 36. Dies kann beispielsweise durch Ausgabe auf einen Datenträger (CD, Chip, Festplatte und dergleichen) erfolgen. Ebenso kann die Ausgabe 36 auch dadurch erfolgen, dass die gewonnene Bestrahlungsplanung unmittelbar in einer Bestrahlungsanlage 1 in eine Bestrahlung umgesetzt wird.

Wird im Überprüfungsschritt 35 dagegen festgestellt, dass noch kein, insbesondere lokales Optimum der Bestrahlungsplanung erreicht wurde, so wird in eine zweite Programmschleife 37 gesprungen und eine neue "Test-Bestrahlungsplanung" durchgerechnet. Die zweite Programmschleife 37 wird so oft durchlaufen, bis eine brauchbare Bestrahlungsplanung ermittelt wurde. Insbesondere ist es auch möglich, dass im Rahmen des Überprüfungsschritts 35 auch unterschiedliche Scanpfade 28 durchiteriert werden.

**Bezugszeichenliste:**

| | | | |
|---|---|---|---|
| 1 | Bestrahlungsanlage | 22 | Pixel |
| 2 | Teilchenstrahl | 23 | Zentralpunkt |
| 3 | Patient | 24 | Rippenbereich |
| 4 | Behandlungsraum | 25 | "Normalgewebe"-Bereich |
| 5 | Liege | 26 | Gewebediskontinuität |
| 6 | Schwerionenbeschleuniger | 27 | Überlappungsbereich |
| 7 | Ionenquelle | 28 | Scanpfad |
| 8 | Linearbeschleuniger | 29 | Rasterpunkt |
| 9 | Synchrotronring | 30 | Verfahren zur Erstellung einer Bestrahlungsplanung |
| 10 | Extraktionsseptum | | |
| 11 | | 31 | einlesen von Daten |
| 12 | Ablenkspulenpaar | 32 | Berechnung Dosiseintrag |
| 13 | passiver Energiemodulator | 33 | erste Programmschleife |
| 14 | keilförmiger Block | 34 | Überprüfung ob Gitter vollständig berechnet |
| 15 | Datenleitung | | |
| 16 | Steuereinheit | 35 | Überprüfung auf Vorliegen von optimierter Bestrahlungsplanung |
| 17 | Mittelpunkt | | |
| 18 | 50%-Linie | 36 | Ausgabe der Bestrahlungsplanung |
| 19 | schraffierter Bereich | | |
| 20 | 10%-Linie | 37 | zweite Programmschleife |
| 21 | Gitter | | |

## Patentansprüche

1. Verfahren (30) zur Erstellung einer Bestrahlungsplanung zur Bestrahlung eines zumindest zeitweise und/oder zumindest bereichsweise bewegten Zielvolumenbereichs (3) mit einer Partikelstrahlerzeugungsvorrichtung (1), bei dem ein Dosiseintrag des Bestrahlungs-Strahls (2) der Partikelstrahlerzeugungsvorrichtung (1) in einem Zielvolumenbereich (3) berücksichtigt wird, und bei dem die Bestrahlung zumindest teilweise durch ein Scan-Verfahren (28) erfolgt, wobei bei der Erstellung der Bestrahlungsplanung zumindest bereichsweise zumindest eine Bestrahlungs-Strahl-Inhomogenität (2, 18, 20) des von der Partikelstrahlerzeugungsvorrichtung bereitgestellten bleistiftdünnen Partikelstrahls, berücksichtigt wird.

2. Verfahren (30) nach Anspruch 1, **dadurch gekennzeichnet, dass** zumindest eine Bestrahlungs-Strahl-Inhomogenität (2, 18, 20) eine Inhomogenität bezüglich der Bestrahlungs-Strahlintensität (18, 20), insbesondere hinsichtlich der ortsaufgelösten Bestrahlungs-Strahlintensität (18, 20) darstellt.

3. Verfahren (30) nach Anspruch 1 oder 2, **dadurch gekennzeichnet, dass** eine Berücksichtigung von Bewegungen zumindest eines Zielvolumenbereichs (3) zumindest zeitweise und/oder zumindest bereichsweise unter Verwendung von Bewegungskompensation (12, 13) erfolgt, insbesondere unter Verwendung von Korrektur-Vektoren für die Ansteuerung der Strahlungserzeugungsvorrichtung (1), insbesondere die Ansteuerung des Bestrahlungs-Strahls (2).

4. Verfahren (30) nach einem der vorangehenden Ansprüche, **dadurch gekennzeichnet, dass** sich die bei der Erstellung der Bestrahlungsplanung verwendete Ortsauflösung, insbesondere die Ortsauflösung des Bestrahlungs-Strahls (2), zumindest zeitweise und/oder zumindest bereichsweise an der Ortsauflösung (22) einer Diagnoseeinrichtung, insbesondere einer bildgebenden Diagnoseeinrichtung orientiert.

5. Verfahren (30) nach einem der vorangehenden Ansprüche, **dadurch gekennzeichnet, dass** es sich bei dem Bestrahlungs-Strahl (2) um einen Protonen-Strahl (2) und/oder um einen Schwerionen-Strahl (2) handelt.

6. Verfahren (30) nach einem der vorangehenden Ansprüche, **dadurch gekennzeichnet, dass** eine Bestrahlung zumindest zeitweise und/oder zumindest teilweise durch ein Raster-Scan-Verfahren (28), durch ein Spot-Scan-Verfahren und/oder durch ein kontinuierliches Scan-Verfahren erfolgt.

7. Verfahren (30) nach einem der vorangehenden Ansprüche, **dadurch gekennzeichnet, dass** zumindest zeitweise und/oder zumindest bereichsweise eine unterschiedliche Gewichtung unterschiedlicher Bereiche (22) des Bestrahlungs-Strahls (2) erfolgt .

8. Verfahren (30) nach einem der vorangehenden Ansprüche, insbesondere Verfahren nach Anspruch 7, **dadurch gekennzeichnet, dass** Überlappungsbereiche (27) bei der Bestrahlung benachbarter Bestrahlungspunkte (29) mit dem Bestrahlungs-Strahl (2) zumindest zeitweise und/oder zumindest bereichsweise gewichtet, insbesondere stärker gewichtet, berücksichtigt werden.

9. Verfahren (30) nach einem der vorangehenden Ansprüche, **dadurch gekennzeichnet, dass** zumindest zeitweise und/oder zumindest bereichsweise eine Gewichtung basierend auf Material-Parametern (24, 25) erfolgt, insbesondere basierend auf ortsvariablen Material-Parametern (24, 25).

10. Verfahren (30) nach einem der vorangehenden Ansprüche, insbesondere nach einem der Ansprüche 6 bis 9, **dadurch gekennzeichnet, dass** die Wegführung des Scan-Pfads (28) im Rahmen der Bestrahlung-Planung (30) zumindest zeitweise und/oder zumindest bereichsweise berücksichtigt wird, insbesondere verändert wird, besonders bevorzugt optimiert wird.

11. Verfahren (30) zur Ansteuerung einer Strahlungserzeugungsvorrichtung (1) zur Applizierung eine ortsaufgelösten Strahlendosis in zumindest einem zumindest zeitweise und/oder zumindest bereichsweise bewegten Zielvolumenbereich (3) unter Verwendung der Partikelstrahlerzeugungsvorrichtung (1), ausgenommen zur Behandlung von Menschen und Tieren, **dadurch gekennzeichnet, dass** zumindest bereichsweise eine Bestrahlungsplanung verwendet wird, welche nach einem Verfahren (30) gemäß einem der Ansprüche 1 bis 10 erstellt wurde.

12. Vorrichtung (16) zur Erstellung einer Bestrahlungsplanung, welche derart ausgebildet und eingerichtet ist, dass diese zumindest zeitweise und/oder zumindest bereichsweise ein Verfahren (30) nach einem der Ansprüche 1 bis 10 durchführt.

13. Vorrichtung (16) zur Ansteuerung zumindest einer Partikelstrahlerzeugungsvorrichtung (1) aufweisend zumindest eine Vorrichtung (16), die derart ausgebildet und eingerichtet ist, dass diese zumindest teilweise ein Verfahren (30) zur Ansteuerung einer Partikelstrahlerzeugungsvorrichtung (1) zur Applizierung einer ortsaufgelösten Strahlendosis in zumindest einem zumindest zeitweise und/oder zumindest bereichsweise bewegten Zielvolumenbereich (3) unter Verwendung der Partikelstrahlerzeugungsvorrichtung (1) durchführt, **dadurch gekennzeichnet, dass** zumindest bereichsweise eine Bestrahlungsplanung verwendet wird, welche nach einem Verfahren (30) gemäß einem der Ansprüche 1 bis 10 erstellt wurde.

## Claims

1. Method (30) for drafting an irradiation plan for irradiating a target volume area (3) which moves at least at some times and/or at least in some areas, with a particle beam generating device (1) where a dosage input of the irradiation beam (2) of the particle beam generating device (1) in a target volume area (3) is taken into account, and where the irradiation is achieved at least in part by a scan method (28), wherein at least in some areas at least one irradiation beam inhomogeneity (2, 18, 20) of the pencil-thin particle beam provided by the particle beam generating device is taken into account during drafting of the irradiation plan.

2. Method (30) according to claim 1, **characterized in that** at least one irradiation beam inhomogeneity (2, 18, 20) represents an inhomogeneity in respect of the irradiation beam intensity (18, 20), in particular in respect of the spatially resolved irradiation beam intensity (18, 20).

3. Method (30) according to claim 1 or 2, **characterized in that** movements of at least one target volume area (3) are taken into account at least at some times and/or at least in some areas using movement compensation (12, 13), in particular using correction vectors for controlling the beam generating device (1), in particular for controlling the irradiation beam (2).

4. Method (30) according to one of the preceding claims, **characterized in that** the spatial resolution used when drafting the irradiation plan, in particular the spatial resolution of the irradiation beam (2), is oriented at least at some times and/or at least in some areas to the spatial resolution (22) of a diagnostic device, in particular of an imaging diagnostic device.

5. Method (30) according to one of the preceding claims, **characterized in that** the irradiation beam (2) is a proton beam (2) and/or a heavy ion beam (2).

6. Method (30) according to one of the preceding claims, **characterized in that** an irradiation is achieved at least at some times and/or at least in part by a raster scan method (28), by a spot scan method and/or by a continuous scan method.

7. Method (30) according to one of the preceding claims, **characterized in that** at least at some times and/or at least in some areas different areas (22) of the irradiation beam (2) are weighted differently.

8. Method (30) according to one of the preceding claims, in particular a method according to claim 7, **characterized in that** overlapping areas (27) during irradiation of adjacent irradiation points (29) with the irradiation beam (2) are taken into account by weighting, in particular by greater weighting, at least at some times and/or at least in some areas.

9. Method (30) according to one of the preceding claims, **characterized in that** weighting at least at some times and/or at least in some areas is based on material parameters (24, 25), in particular based on locally variable material parameters (24, 25).

10. Method (30) according to one of the preceding claims, in particular according to one of claims 6 to 9, **characterized in that** during irradiation planning (30) the routing of the scan path (28) at least at some times and/or at least in some areas is taken into account, in particular is altered, particularly preferably is optimized.

11. Method (30) for controlling a beam generating device (1) for application of a spatially resolved radiation dose using the particle beam generating device (1) in at least one target volume area (3) which moves at least at some times and/or at least in some areas, except for the treatment of humans and animals, **characterized in that** at least in some areas an irradiation plan is used which was drafted using a method (30) according to one of claims 1 to 10.

12. Device (16) for drafting an irradiation plan which is designed and configured such that it implements at least at some times and/or at least in some areas a method (30) according to one of claims 1 to 10.

13. Device (16) for controlling at least one particle beam generating device (1) having at least one device (16) which is designed and configured such that it implements at least in part a method (30) for controlling a particle beam generating device (1) for application of a spatially resolved radiation dose using the particle beam generating device (1) in at least one target volume area (3) moved at least at some times and/or at least in some areas, **characterized in that** at least in some areas an irradiation plan is used which was drafted using a method (30) according to one of claims 1 to 10.

## Revendications

1. Procédé (30) pour établir un programme d'irradiation afin d'irradier à l'aide d'un dispositif générateur de rayonnement corpusculaire (1) une zone de volume cible (3) en mouvement au moins par intermittence et/ou au moins partiellement, procédé dans lequel une application de dose du faisceau d'irradiation (2) du dispositif générateur de rayonnement corpusculaire (1) dans un zone de volume cible (3) est prise en compte, et dans lequel l'irradiation est effectuée au moins partiellement au moyen d'un procédé de balayage (28), sachant que lors de l'établissement du programme d'irradiation est prise en compte au moins partiellement au moins une hétérogénéité du faisceau d'irradiation (2, 18, 20) du rayonnement corpusculaire ayant l'épaisseur d'un crayon et fourni par le dispositif générateur de rayonnement corpusculaire.

2. Procédé (30) selon la revendication 1, **caractérisé en ce qu'**au moins une hétérogénéité du faisceau d'irradiation (2, 18, 20) représente une hétérogénéité concernant l'intensité du faisceau d'irradiation (18, 20), en particulier concernant l'intensité du faisceau d'irradiation (18, 20) spatialement résolue.

3. Procédé (30) selon la revendication 1 ou 2, **caractérisé en ce qu'**a lieu une prise en compte de mouvements d'au moins une zone du volume cible (3) au moins par intermittence et/ou au moins partiellement en utilisant une compensation de mouvement (12, 13), en particulier en utilisant des vecteurs de correction pour l'activation du dispositif générateur de rayonnement (1), en particulier l'activation du faisceau d'irradiation (2).

4. Procédé (30) selon l'une des revendications précédentes, **caractérisé en ce que** la résolution spatiale utilisée lors de l'établissement du programme d'irradiation, en particulier la résolution spatiale du faisceau d'irradiation (2), s'oriente au moins par intermittence et/ou au moins partiellement sur la résolution spatiale (22) d'un dispositif de diagnostic, en particulier d'un dispositif de diagnostic par imagerie.

5. Procédé (30) selon l'une des revendications précédentes, **caractérisé en ce que** le faisceau d'irradiation (2) est un faisceau de protons (2) et/ou un faisceau d'ions lourds (2).

6. Procédé (30) selon l'une des revendications précédentes, **caractérisé en ce qu'**une irradiation est effectuée au moins par intermittence et/ou au moins partiellement par un procédé de balayage tramé (28), par un procédé de balayage ponctuel et/ou par un procédé de balayage continu.

7. Procédé (30) selon l'une des revendications précédentes, **caractérisé en ce qu'**au moins par intermittence et/ou au moins partiellement a lieu une pondération différente de différentes zones (22) du faisceau d'irradiation (2).

8. Procédé (30) selon l'une des revendications précédentes, en particulier procédé selon la revendication 7, **caractérisé en ce que** lors de l'irradiation de points d'irradiation (29) voisins avec le faisceau d'irradiation (2) sont prises en compte des zones de chevauchement (27) pondérées au moins par intermittence et/ou au moins partiellement, en particulier plus fortement pondérées.

9. Procédé (30) selon l'une des revendications précédentes, **caractérisé en ce qu'**au moins par intermittence et/ou au moins partiellement a lieu une pondération se basant sur des paramètres de matériau (24, 25), en particulier se basant sur des paramètres de matériau (24, 25) localement variables.

10. Procédé (30) selon l'une des revendications précédentes, en particulier selon l'une des revendications 6 à 9, **caractérisé en ce que** le tracé du chemin de balayage (28) dans le cadre du programme d'irradiation (30) est pris en compte au moins par intermittence et/ou au moins partiellement, et est en particulier modifiid, notamment de préférence optimisé.

11. Procédé (30) de pilotage d'un dispositif générateur de rayonnement (1) pour appliquer une dose d'irradiation spatialement résolue dans au moins une zone de volume cible (3) en mouvement au moins par intermittence et/ou au moins partiellement, en utilisant le dispositif générateur de rayonnement corpusculaire (1), à l'exclusion d'un traitement d'êtres humains et d'animaux, **caractérisé en ce qu'**est mis en ouvre au moins par zones un programme d'irradiation qui a été établi d'après un procédé (30) selon l'une des revendications 1 à 10.

12. Dispositif (16) pour établir un programme d'irradiation et conçu et aménagé de telle manière qu'il réalise au moins par intermittence et/ou au moins par zones un procédé (30) selon l'une des revendications 1 à 10.

13. Dispositif (16) pour piloter au moins un dispositif générateur de rayonnement corpusculaire (1), présentant au moins un dispositif (16) conçu et aménagé de telle manière qu'il réalise au moins partiellement un procédé (30) pour piloter un dispositif générateur de rayonnement corpusculaire (1) destiné à appliquer une dose d'irradiation spatialement résolue dans au moins une zone de volume cible (3) en mouvement au moins par intermittence et/ou au moins partiellement, en utilisant le dispositif générateur de rayonnement corpusculaire (1), **caractérisé en ce qu'**est mis en oeuvre au moins par zones un programme d'irradiation qui a été établi d'après un procédé (30) selon l'une des revendications 1 à 10.
